(19) Europäisches Patentamt
European Patent Office
Office européen des brevets

(11) Publication number: **0 122 037**
**B1**

(12) **EUROPEAN PATENT SPECIFICATION**

(45) Date of publication of patent specification: **19.08.87**

(21) Application number: **84301548.8**

(22) Date of filing: **08.03.84**

(51) Int. Cl.⁴: **C 07 C 79/46,** C 07 C 69/92, C 07 C 67/14, C 07 C 76/02, A 01 N 43/00, C 07 D 231/10, C 07 C 131/00, C 07 C 103/76, C 07 D 307/12, C 07 C 103/84, A 01 N 37/10

(54) **Substituted diphenylether compounds useful as herbicides.**

(30) Priority: **07.04.83 GB 8309397**
**11.04.83 GB 8309773**

(43) Date of publication of application:
**17.10.84 Bulletin 84/42**

(45) Publication of the grant of the patent:
**19.08.87 Bulletin 87/34**

(84) Designated Contracting States:
**BE CH DE FR GB IT LI NL SE**

(56) References cited:
**EP-A-0 003 416**
**EP-A-0 021 692**
**EP-A-0 034 402**
**EP-A-0 063 741**

**The file contains technical information submitted after the application was filed and not included in this specification**

(73) Proprietor: **IMPERIAL CHEMICAL INDUSTRIES PLC**
**Imperial Chemical House Millbank**
**London SW1P 3JF (GB)**

(72) Inventor: **Cartwright, David, Dr.**
**1 Stonehaven Drive**
**Woodley Reading Berkshire (GB)**
Inventor: **Collins, David John, Dr.**
**10 Ardwell Close**
**Crowthorne Berkshire (GB)**
Inventor: **Griffin, David Alan, Dr.**
**12 Orion Roman Hill**
**Bracknell Berkshire (GB)**

(74) Representative: **Downes, John Edward et al**
**Imperial Chemical Industries PLC Legal**
**Department: Patents Po Box 6**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 122 037 B1

**Description**

This invention relates to substituted diphenyl ether compounds useful as herbicides, to processes of making them, and to herbicidal compositions and processes utilising them.

A large number of substituted diphenyl ether compounds have been proposed for use as herbicides. By way of examples of the many publications containing such proposals, there may be mentioned as an early example UK Patent 974475, and as more recent examples US Patents 3784635; 3979437; 3928416 and European Patent Applications with publication numbers 20052, 21692, 23100, 40898, 63741, 66217 and 70990.

European Patent Application with Publication No. 34402 discloses compounds of formula A

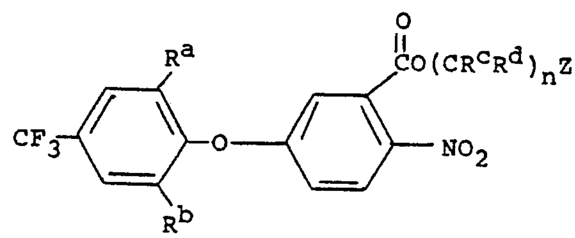

A

wherein $R^a$ is hydrogen, halo, trihalomethyl, alkyl, cyano or nitro; $R^b$ is hydrogen, halo or trifluoromethyl; $R^c$ and $R^d$ are the same or different and are selected from hydrogen, $(C_{1-4})$alkyl, phenyl and benzyl, the $CR^cR^d$ groups being the same or different when n is $>1$; n is an integer of from 1 to 5 and Z is halo, nitro, cyano, trihalomethyl, methylthio, methylsulphioyl, methylsulphonyl, acetyl, phenyl, or a heterocyclic group of from 4 to 6 atoms, from 1 to 4 of which are selected from nitrogen, oxygen or sulphur.

The Applicants have found a class of herbicides, which partially overlap with formula A above.

According to the present invention there are provided diphenyl ether compounds of the formula (I):

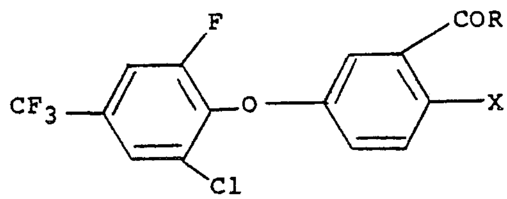

(I)

wherein X is a nitro group or a chlorine atom and R is:

(a) a group $ZR^3$ wherein Z is oxygen or sulphur, and $R^3$ is an aliphatic or alicyclic radical (e.g. an aliphatic or alicyclic radical of 1 to 6 carbon atoms) bearing one or more of the following substituents: halogen; nitro; hydroxy; $CF_3$; alkoxy (e.g. of 1 to 4 carbon atoms); alkylthio, alkylsulphinyl, or alkylsulphonyl each having for example from 1 to 4 carbon atoms; phenyl; heterocyclyl containing for example 5 or 6 ring atoms; a group $-NR^1R^2$ as defined below; cyano; carboxy or a salt thereof; alkoxycarbonyl (e.g. having from 2 to 5 carbon atoms); a group $-CONR^1R^2$ wherein $-NR^1R^2$ is as defined below; formyl; alkanoyl having for example 2 to 5 carbon atoms; or a 1-pyrazolyl, 1-imidazolyl, 1-(1,2,4-triazolyl) or 2-(1,2,3-triazolyl) group optionally bearing one or more halogen, methyl, methoxy, or methylthio substituents.

(b) a group $-NR^1R^2$ wherein $R^1$ is hydrogen, an optionally substituted aliphatic or alicyclic radical (eg. an aliphatic radical of 1 to 6 carbon atoms or an alicyclic radical of 3 to 6 carbon atoms) or an optionally substituted phenyl radical, and $R^2$ is defined as for $R^1$ but may additionally represent hydroxy; alkoxy of 1 to 4 carbon atoms; alkoxy of 1 to 4 carbon atoms substituted by phenyl or by carboxy or a salt, ester, or amide thereof; or alkoxy of 1 to 4 carbon atoms substituted by amino or mono- or di-dialkylamino wherein the alkyl groups have for example from 1 to 4 carbon atoms; or wherein the group $-NR^1R^2$ is constituted by an optionally methyl-substituted pyrrolidine, piperidine, or morpholine ring, or by a succinimido, or phthalimido radical or by a group of the formula:

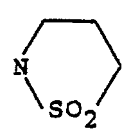 or 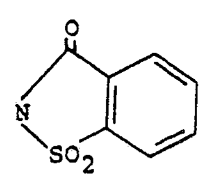

or

(c) a group $-NS^1S^2$ wherein $S^1$ is hydrogen, an alkyl group (eg. $C_1$ to $C_4$ alkyl), an agriculturally acceptable cation, or chlorine, bromine, or iodine; and $S^2$ is a group

$$-\underset{\underset{O}{\|}}{C}OS^3$$

wherein $S^3$ is an optionally substituted aliphatic or alicyclic radical (eg. having from 1 to 6 carbon atoms), or an optionally substituted phenyl radical; or $S^2$ is a group

$$\begin{array}{c} S^9 \quad S^4 \\ \diagdown \; \diagup \\ -(C)_n-S^5 \end{array}$$

wherein $S^4$ and $S^9$ are independently hydrogen or a $C_1$ to $C_4$ alkyl group or phenyl group and n is 1, 2, or 3, and $S^5$ is a CN group, a group $-CONS^6S^7$ wherein each of $S^6$ and $S^7$ is hydrogen, an optionally substituted aliphatic or alicyclic radical (eg. having from 1 to 6 carbon atoms) or an optionally substituted phenyl radical; or $S^5$ is a group

$$\begin{array}{l} -COS^8 \\ \;\;\; \| \\ \;\;\; O \end{array}$$

wherein $S^8$ is hydrogen, an agriculturally acceptable cation, or an optionally substituted aliphatic or alicyclic radical (having for example from 1 to 6 carbon atoms) or an optionally substituted phenyl radical, or

(d) an optionally substituted phenoxy, phenylthio, or 2-sulpholanoxy group, or a group $-ON{=}CR^4R^5$ wherein $R^4$ is hydrogen, halogen, alkyl, mononuclear aryl, $(C_1-C_6)$alkoxy, $(C_1-C_6)$alkylthio or dialkylamino; and $R^5$ is substituted or unsubstituted alkyl, trifluoromethyl, cyano, carboxy, substituted or unsubstituted alkoxy, alkoxycarbonyl, alkylcarbonyl, alkylthio, aminocarbonyl, mononuclear arylcarbonyl, mononuclear aryloxy or carboxy including agronomically acceptable amides, esters or salts of said carboxy; or $R^4$ and $R^5$ may be joined together to form a ring containing from 4 to 8 nuclear carbon atoms.

The term halogen in the foregoing definition is intended to include fluorine, chlorine, bromine, and iodine. The term aliphatic is intended to include alkyl, alkenyl, and alkynyl radicals, whether straight chain or branched, and corresponding radicals with more than one double or triple bond. It is also intended to include radicals which comprise an alkyl, alkenyl, or alkynyl radical bearing an alicyclic radical as a substituent. The term alicyclic radical includes for example cycloalkyl radicals, for example cyclopropyl and cyclohexyl. When $R^1$ or $R^2$ is an aliphatic or alicyclic radical, possible substituents include, for example, halogen; hydroxy; alkoxy (eg. of 1 to 4 carbon atoms); carboxy or a salt ester or amide thereof; cyano; phenyl; or heterocyclyl of 5 to 7 ring atoms. When R is phenoxy, or $R^1$ or $R^2$ is a phenyl or heterocyclyl radical, examples of possible substituents include those recited above for the case when one of $R^1$ or $R^2$ is aliphatic or alicyclic, and further include alkyl (eg. of 1 to 4 carbon atoms), nitro, $CF_3$, and alkylthio, alkylsulphinyl, and alkylsulphonyl, each of 1 to 4 carbon atoms.

Where $S^3$, $S^6$, $S^7$, or $S^8$ is an aliphatic radical it may be for example an alkyl, alkenyl, or alkynyl radical having for example from 1 to 6 carbon atoms, and it may be straight or branched. When $S^3$, $S^6$, $S^7$ or $S^8$ is an alicyclic radical it may for example contain from 3 to 6 carbon atoms; it may be, for example, cyclohexyl.

Examples of substituents which may be present in a radical $S^3$, $S^6$, $S^7$ or $S^8$ when it is an aliphatic radical include halogen (ie. fluorine, chlorine, bromine, or iodine), hydroxy, alkoxy (eg. of 1 to 4 carbon atoms) and phenyl. Examples of substituents which may be present in a radical $S^3$, $S^6$, $S^7$ or $S^8$ when it is a phenyl group include nitro, cyano, methyl, $CF_3$, alkoxy (eg. $C_{1-4}$), hydroxy, and halogen.

Where the foregoing definition includes reference to salts, esters, or amides of a carboxy group, the salts referred to are formed from an agriculturally acceptable cation, for example alkali metal, alkaline earth metal, ammonium, or substituted ammonium cations; for example from sodium, potassium, lithium, calcium, or magnesium cations. Substituted ammonium cations include, for example those in which one, two, three or four of the hydrogen atoms in the ammonium cations are replaced by an aliphatic radical having for example from 1 to 6 carbon atoms and optionally being substituted, for example by one or more hydroxy or phenyl radicals. Esters of the said carboxy groups include esters formed from aliphatic and alicyclic alcohols. Amides include those formed from ammonia or monoalkyl or dialkylamines wherein the alkyl groups each contain from 1 to 6 carbon atoms.

Particular examples of compounds falling within the scope of the invention are listed in Table I below:

TABLE 1

| Compound No. | X | R |
|---|---|---|
| 1 | $NO_2$ | $-OCH_2CH_2Cl$ |
| 2 | Cl | $-OCH_2CH_2Cl$ |
| 3 | $NO_2$ | $-OCH_2CH_2NO_2$ |
| 4 | Cl | $-OCH_2CH_2NO_2$ |
| 5 | $NO_2$ | 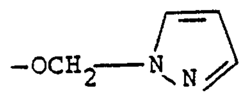 |
| 6 | Cl | 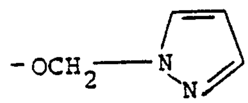 |
| 7 | $NO_2$ | 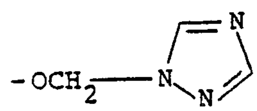 |
| 8 | Cl | 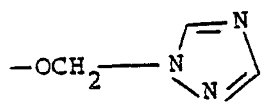 |
| 9 | $NO_2$ | 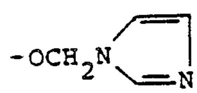 |
| 10 | Cl | 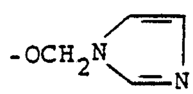 |
| 11 | $NO_2$ | 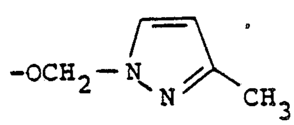 |
| 12 | Cl | 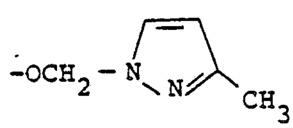 |
| 13 | $NO_2$ | 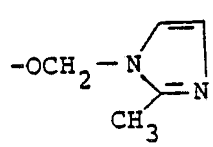 |

TABLE 1 continued

| Compound No. | X | R |
|---|---|---|
| 14 | Cl | 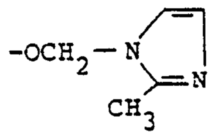 |
| 15 | $NO_2$ | 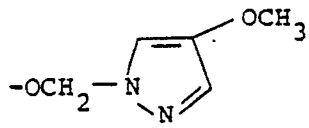 |
| 16 | Cl | 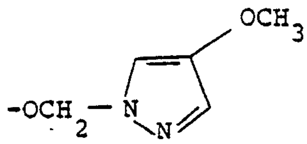 |
| 17 | $NO_2$ | 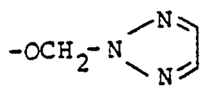 |
| 18 | Cl | 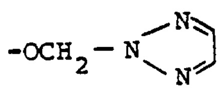 |
| 19 | $NO_2$ | 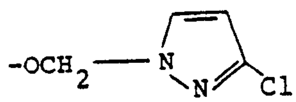 |
| 20 | Cl | 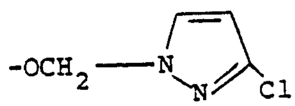 |
| 21 | $NO_2$ | 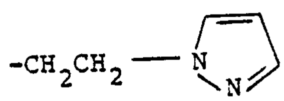 |
| 22 | Cl | 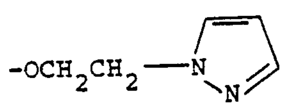 |
| 23 | $NO_2$ | 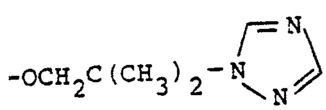 |
| 24 | Cl | 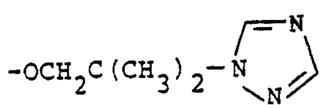 |
| 25 | $NO_2$ | $—OCH_2CO_2H$ |
| 26 | Cl | $—OCH_2CO_2H$ |

TABLE 1 continued

| Compound No. | X | R |
|---|---|---|
| 27 | $NO_2$ | $—OCH(CH_3)CO_2C_2H_5$ |
| 28 | Cl | $—OCH(CH_3)CO_2C_2H_5$ |
| 29 | $NO_2$ | $—OC_6H_5$ |
| 30 | Cl | $—OC_6H_5$ |
| 31 | $NO_2$ | $2\text{-}NO_2.C_6H_4.O—$ |
| 32 | Cl | $2\text{-}NO_2.C_6H_4.O–$ |
| 33 | $NO_2$ | 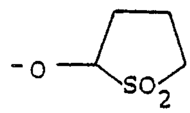 |
| 34 | Cl | 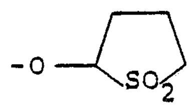 |
| 35 | $NO_2$ | $—NH_2$ |
| 36 | Cl | $—NH_2$ |
| 37 | $NO_2$ | $—ON{=}C(CH_3)_2$ |
| 38 | Cl | $—ON{=}C(CH_3)_2$ |
| 39 | $NO_2$ | $—NHCH_3$ |
| 40 | Cl | $—NHCH_3$ |
| 41 | $NO_2$ | 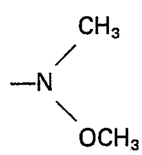 |
| 42 | Cl | 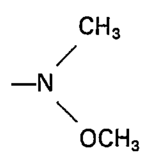 |
| 43 | $NO_2$ | $—NHOCH_2CO_2H$ |
| 44 | Cl | $—NHOCH_2CO_2H$ |
| 45 | $NO_2$ | $—NHOCH_2CO_2C_2H_5$ |
| 46 | Cl | $—NHOCH_2CO_2C_2H_5$ |
| 47 | $NO_2$ | 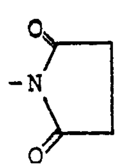 |

TABLE 1 continued

| Compound No. | X | R |
|---|---|---|
| 48 | Cl | 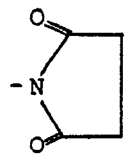 |
| 49 | $NO_2$ | 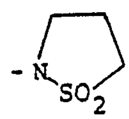 |
| 50 | Cl | 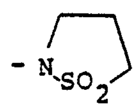 |
| 51 | $NO_2$ | 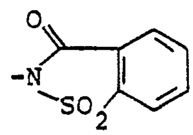 |
| 52 | Cl | 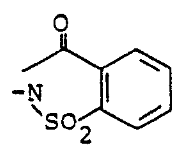 |
| 53 | $NO_2$ | 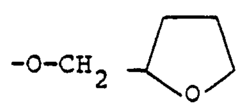 |
| 54 | Cl | 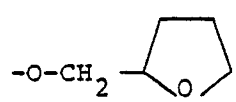 |
| 55 | $NO_2$ | $—NHCH_2CO_2C_2H_5$ |
| 56 | Cl | $—NHCH_2CO_2C_2H_5$ |
| 57 | $NO_2$ | $—NHOCH_3$ |
| 58 | Cl | $—NHOCH_3$ |
| 59 | $NO_2$ | $CH_3$<br>$—ON{=}C—CO_2C_2H_5$ |
| 60 | Cl | $CH_3$<br>$—ON{=}C—CO_2C_2H_5$ |

TABLE 1 continued

| Compound No. | X | R |
|---|---|---|
| 61 | $NO_2$ | $CH_3$<br>\|<br>$—NHCHCO_2C_2H_5$ |
| 62 | Cl | $CH_3$<br>\|<br>$—NHCHCO_2C_2H_5$ |
| 63 | $NO_2$ | $—NHCO_2CH_3$ |
| 64 | $NO_2$ | $—NHCH_2CN$ |
| 65 | $NO_2$ | $—NHCH_2CONH_2$ |
| 66 | $NO_2$ | $OCH_2CO_2Et$ |
| 67 | Cl | $OCH_2CO_2Et$ |

Where a compound falling within the scope of formula (I) above is capable of existing in optically isomeric forms, the invention includes the separate D and L forms of the compounds as well as mixtures of the D and L forms in all proportions. As normally prepared by chemical synthesis, such optically isomeric compounds will normally be obtained as mixtures of equal proportions of the D and L forms (ie. racemic mixtures).

The compounds of the invention may be prepared in various ways, for example by the process outlined in Scheme A below:

Scheme A

(a) 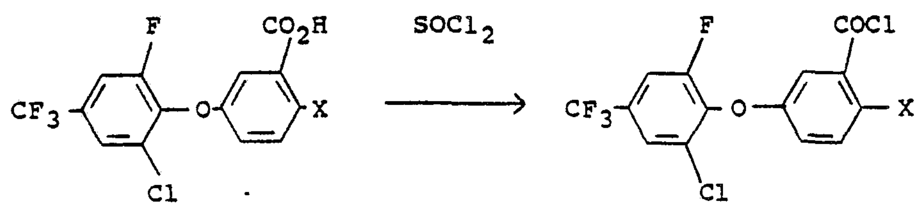


(II)

(b) (III) + RH $\xrightarrow[\text{acceptor}]{\text{acid}}$ (I)

In Scheme A, the symbols R and X have the meanings previously assigned to them. The carboxylic acids (II) required as starting materials are known compounds. The other reactants RH are also in general known compounds or, where new in themselves, may be prepared by methods known for the preparation of the analogous known compounds. According to Scheme A, a carboxylic acid (II) is treated with thionyl chloride to convert it to the acid chloride (III). Usually the reaction may be carried out by heating the carboxylic acid (II) under reflux with excess of thionyl chloride and removing the excess of thionyl chloride under reduced pressure when reaction is complete. The acid is then reacted with the compound RH, preferably in a solvent or diluent inert towards the reactants, and preferably in the presence of an acid acceptor.

Depending upon the particular reactant RH chosen for use, the reaction mixture may be heated to accelerate the reaction, for example to a temperature in the range from 30—130°C or above. In some cases, it may be desirable to cool the reaction mixture at the start in order to moderate a vigorous exothermic reaction. The solvent or diluent may be for example a liquid hydrocarbon, for example diethyl ether or tetrahydrofuran; or a ketone, for example methyl ethyl ketone. Other solvents and diluents will be known to those skilled in the art; the choice of solvent or diluent is not critical. The acid acceptor is preferably present in at least an equimolar amount with respect to the reactants. Examples of acid acceptors include tertiary amines, for example pyridine, dimethylaniline, and triethylamine and inorganic bases, for example sodium or potassium carbonate. The product may be isolated from the reaction by conventional methods.

An alternative method of preparing the compounds of the invention is outlined in Scheme B below:

Scheme B

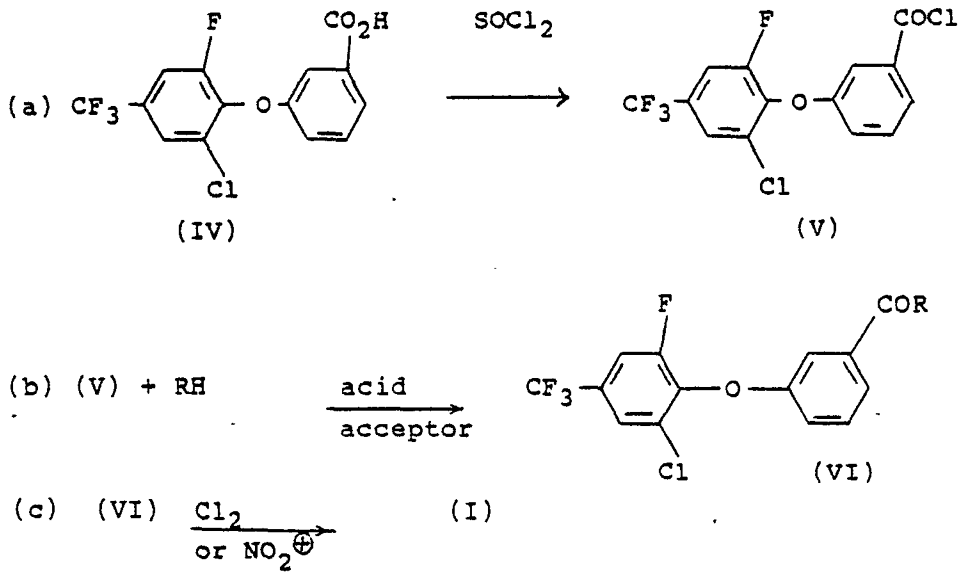


According to Scheme B, the carboxylic acid (IV) is converted to the corresponding acid chloride (V) as described in Scheme A. The acid chloride is then reacted with the appropriate intermediate RH also as described in Scheme A. The product (VI) so obtained is then converted to the required compound (I) by nitration or chlorination. Thus, where X is to be a nitro group, the diphenyl ether (VI) is treated with a nitrating agent, for example a mixture of nitric and sulphuric acids, or potassium nitrate/sulphuric acid. Where X is to be chlorine, the diphenyl ether (VI) is treated with a chlorinating agent; for example, the diphenyl ether may be dissolved in a solvent (eg. glacial acetic acid) and treated with gaseous chlorine. In either case the required compound (I) is isolated from the reaction mixture by conventional methods.

Compounds of the invention wherein R is —$NS^1S^2$ may also be prepared in a variety of ways. A process for preparing compounds in which $S^1$ is hydrogen and $S^2$ is a group

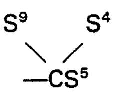

is outlined in Scheme C below:

Scheme C

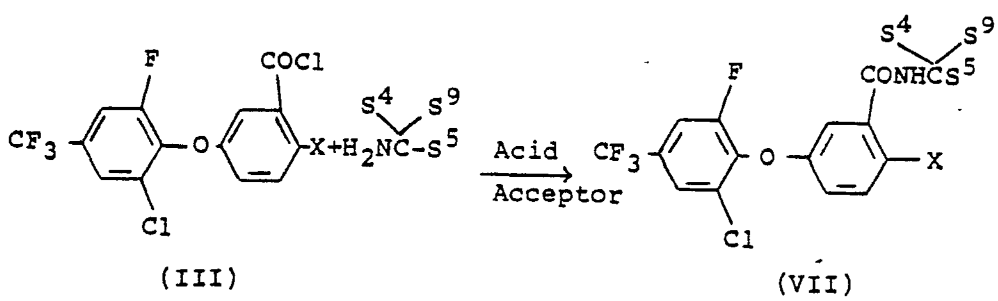


The amines

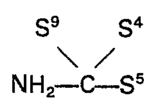

are in general known compounds. According to Scheme C, an acid chloride (II) is reacted with such an amine, preferably in a solvent or diluent inert towards the reactants, and preferably in the presence of an acid acceptor.

Depending upon the particular reactants chosen for use, the reaction mixture may be heated to accelerate the reaction, for example to a temperature in the range from 30—130°C or above. In some cases,

it may be desirable to cool the reaction mixture at the start in order to moderate a vigorous exothermic reaction. The solvent or diluent may be for example a liquid hydrocarbon, for example diethyl ether or tetrahydrofuran; or a ketone, for example methyl ethyl ketone. Other solvents and diluents will be known to those skilled in the art; the choice of solvent or diluent is not critical. The acid acceptor is preferably present in at least an equimolar amount with respect to the reactants. Examples of acid acceptors include tertiary amines, for example pyridine, dimethylaniline, and triethylamine and inorganic bases, for example sodium or potassium carbonate. The product may be isolated from the reaction by conventional methods.

Compounds of the invention wherein $S^1$ is an agriculturally acceptable cation may be prepared from compounds of formula (VII) by treatment of the latter with an equimolar amount of an alkali metal or alkaline earth metal hydroxide or carbonate, or with ammonium hydroxide or a substituted ammonium hydroxide.

Compounds of the invention wherein $S^1$ is a chlorine, bromine, or iodine atom may be prepared by reacting an alkali metal hypohalite (eg. a sodium hypohalite for example sodium hypochlorite) with a compound of the formula (VII).

The reaction is generally carried out in aqueous or aqueous-organic solution or suspension, in the presence of a water-miscible solvent such as an alcohol or ketone, for example methanol, ethanol, acetone, methyl ethyl ketone or methyl isobutyl ketone. The reaction is generally carried out at a temperature from −10 to +60°C preferably from 10 to 40°C. The concentration of starting material in the reaction medium is preferably from 0.5 to 30% by weight, preferably from 2% to 10%. The amount of hypohalite used is generally from 1 to 3 times the stoichiometric amount.

Compounds according to the invention wherein $S^1$ is hydrogen and $S^2$ is a group

$$-\underset{\underset{O}{\|}}{C}OS^3$$

may be prepared for example by the process outlined in Scheme D.

Scheme D

(a)

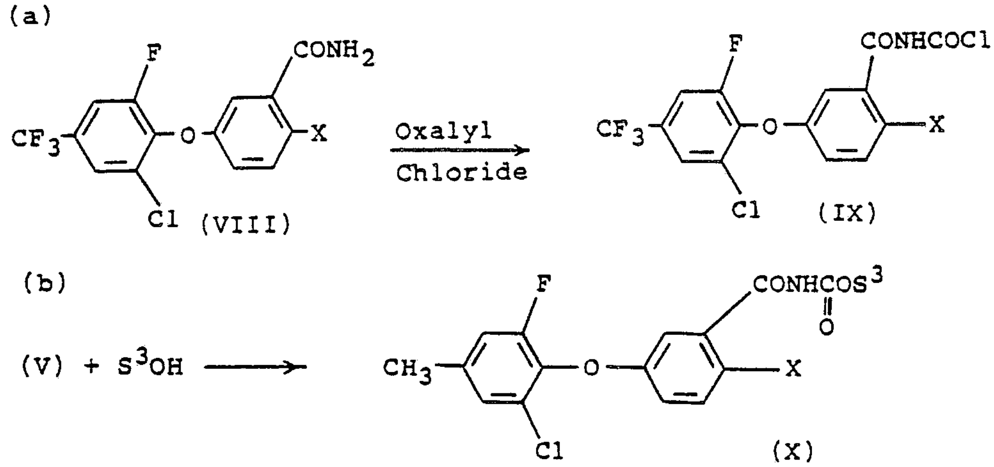


(VIII) (IX)

(b)

(X)

The substituted benzamide derivatives (VIII) used in Scheme D are either known compounds or can be prepared by standard methods from the corresponding known carboxylic acids. In stage (a) of Scheme D, a substituted benzamide (VIII) is reacted with oxalyl chloride to give an acid chloride (IX), preferably in a solvent or diluent inert toward the reactants. The reaction is preferably accelerated by heating, for example to a temperature in the range from 50 to 120°. The acid chloride derivative, after removal of the solvent, is then reacted with an alcohol or phenol $S^3OH$ to give the carbamate derivative (X). Conveniently this may be done by heating the acid chloride (IX) with an excess of the alcohol or phenol, for example at a temperature in the range from 50 to 100° or more, the excess of the reactant $S^3OH$ acting as a solvent. The compounds (X) may be converted into salts by reaction with an alkali or alkaline earth metal hydroxide or carbonate or with ammonium or substituted ammonium cation as described above. The N-chloro, bromo, or iodo derivatives may also be prepared as described above.

In the above schemes, the substituent X is shown as being present throughout the reaction sequence; however, in alternative processes, the X-substituent is introduced by nitration or chlorination as the last step of the process, X being hydrogen in the intermediates.

The compounds of the invention are useful as herbicides. In another aspect, therefore, the invention provides a process of inhibiting the growth of unwanted plants, which comprises applying to the plants, or to the locus thereof, a phytotoxic amount of a compound of the formula (I) as hereinbefore defined. The amount of the compound to be applied in the process may vary, depending upon the particular compound chosen, and the identity of the plant species whose growth is to be inhibited, but in general amounts from 0.1 to 10 kilograms per hectare will be suitable; in many cases from 0.25 to 1.0 kilograms per hectare will be appropriate. The skilled worker in the herbicide art will readily be able to establish appropriate application rates by standard procedures without undue experimentation.

The compounds of the invention are effective in controlling a variety of unwanted plants. The compounds may be applied to the above-ground parts of unwanted plants (post-emergence application) or they may be applied to the soil to prevent the growth of plants from seeds present in the soil (pre-emergence application). The compounds may be used, for example, for selective weed control in crops of soya bean.

The compounds used in the process of the invention are preferably applied in the form of a composition, in which the active ingredient is mixed with a carrier comprising a solid or liquid diluent. In another aspect, therefore, the invention provides a herbicidal composition, comprising as an active ingredient a compound of the formula (I) as hereinbefore defined, in admixture with a solid or liquid diluent. Preferably the composition also comprises a surface-active agent.

The solid compositions of the invention may be for example, in the form of dusting powders, or may take the form of granules. Suitable solid diluents include, for example, kaolin, bentonite, kieselguhr, dolomite, calcium carbonate, talc, powdered magnesia, and Fuller's earth.

Solid compositions may also be in the form of dispersible powders or grains comprising in addition to the active ingredient, a wetting agent to facilitate the dispersion of the powder or grains in liquids. Such powders or grains may include fillers, suspending agents and the like.

Liquid compositions include aqueous solutions, dispersions and emulsions containing the active ingredient preferably in the presence of one or more surface active agents. Water or organic liquids may be used to prepare solutions, dispersions, or emulsions of the active ingredient. The liquid compositions of the invention may also contain one or more corrosion inhibitors for example lauryl isoquinolinium bromide.

Surface active agents may be of the cationic, anionic or non-ionic type. Suitable agents of the cationic type include for example quaternary ammonium compounds, for example cetyltrimethyl ammonium bromide. Suitable agents of the anionic type include for example soaps, salts of aliphatic mono-esters of sulphuric acid, for example sodium lauryl sulphate; and salts of sulphonated aromatic compounds, for example dodecyl-benzenesulphonate, sodium, calcium and ammonium ligno-sulphonate, butyl-naphthalene sulphonate, and a mixture of the sodium salts of diisopropyl- and triisopropyl-naphthalenesulphonic acid. Suitable agents of the non-ionic type include, for example, the condensation products of ethylene oxide with fatty alcohols such as oleyl alcohol and cetyl alcohol, or with alkyl phenols such as octylphenol, nonylphenol, and octylcresol. Other non-ionic agents are the partial esters derived from long chain fatty acids and hexitol anhydrides, for example sorbitol monolaurate; the condensation products of the said partial esters with ethylene oxide and the lecithins.

The compositions which are to be used in the form of aqueous solutions, dispersions or emulsions are generally supplied in the form of concentrate containing a high proportion of the active ingredient, the concentrate being diluted with water before use. These concentrates are usually required to withstand storage for prolonged periods and after such storage to be capable of dilution with water in order to form aqueous preparations which remain homogeneous for a sufficient time to enable them to be applied by conventional spray equipment.

The compositions of the invention may contain, in addition to carriers and surface-active agents, various other constituents to increase their usefulness. They may contain, for example, buffering salts to maintain the pH of the composition within a desired range; antifreeze agents, for example urea or propylene glycol; adjuvants, for example, oils and humectants; and sequestrants, for example citric acid and ethylenediaminetetracetic acid, which help to prevent the formation of insoluble precipitates when the compositions are diluted with hard water. Aqueous dispersions may contain anti-settling agents and anti-caking agents.

The compositions may in general contain a dye or pigment to impart a characteristic colour. Agents for increasing viscosity may be added to reduce the formation of fine droplets during spraying, and thereby reduce spray drift. Other additives useful for particular purposes will be known to those skilled in the formulation art.

In general concentrates may conveniently contain from 10 to 85% and preferably from 25 to 60% by weight of active ingredient. Dilute preparations ready for use may contain varying amounts of the active ingredient, depending upon the purpose for which they are to be used; however, dilute preparations suitable for many uses contain between 0.01% and 10% and preferably between 0.1% and 1% by weight of the active ingredient.

The compounds of the invention can be used in association (for example in the form of a mixture) with another herbicide.

Examples of such herbicides are:

A. benzo-2,1,3-thiadiazin-4-one-2,2-dioxides such as 3-isopropylbenzo-2,1,3-thiadiazin-4-one-2,2-dioxide (bentazon);

B. hormone herbicides, particularly the phenoxy alkanoic acids such as 4-chloro-2-methylphenoxy acetic acid (MCPA), 2-(2,4-dichlorophenoxy)propionic acid (dichlorprop), 2,4,5-trichlorophenoxyacetic acid (2,4,5-T), 4-(4-chloro-2-methylphenoxy)butyric acid (MCPB), 2,4-dichlorophenoxyacetic acid (2,4-D), 4-(2,4-dichlorophenoxy)butyric acid (2,4-DB), 2-(4-chloro-2-methylphenoxy)propionic acid (mecoprop), and their derivatives (eg. salts, esters and amides);

C. 3-[4-(4-halophenoxy)phenyl]-1,1-dialkylureas such as 3-[4-(4-chlorophenoxy)phenyl]-1,1-dimethylurea (chloroxuron);

D. dinitrophenols and their derivatives (e.g. acetates) such as 2-methyl-4,6-dinitrophenol (DNOC), 2-t-butyl-4,6-dinitrophenol (dinoterb), 2-secbutyl-4,6-dinitrophenol (dinoseb) and its ester, dinoseb acetate;

E. dinitroaniline herbicides such as N',N'-diethyl-2,6-dinitro-4-trifluoromethyl-*m*-phenylenediamine (dinitramine), 2,6-dinitro-N,N-dipropyl-4-trifluoromethylaniline (trifluralin) and 4-methysulphonyl-2,6-dinitro-N,N-dipropylaniline (nitralin);

F. phenylurea herbicides such as N'-(3,4-dichlorophenyl)-N,N-dimethylurea (diuron) and N,N-dimethyl-N'-[3-(trifluoromethyl)phenyl]urea (flumeturon); N,N-dimethyl-N'-[4-isopropylphenyl]urea (isoproturon); N,N-dimethyl-N'-[-4-chloro-3-methylphenyl]urea (chortoluron).

G. phenylcarbamoyloxyphenylcarbamates such as 3-[methoxycarbonylamino]phenyl (3-methylphenyl)-carbamate (phenmedipham) and 3-[ethoxycarbonylamino]phenyl phenylcarbamate (desmedipham);

H. 2-phenylpyridazin-3-ones such as 5-amino-4-chloro-2-phenylpyridazin-3-one (pyrazon);

I. uracil herbicides such as 3-cyclohexyl-5,6-trimethyleneuracil (lenacil), 5-bromo-3-sec-butyl-6-methyluracil (bromacil) and 3-t-butyl-5-chloro-6-methyluracil (terbacil);

J. triazine herbicides such as 2-chloro-4-ethylamino-6-(i-propylamino)-1,3,5-triazine (atrazine), 2-chloro-4,6-di(ethylamino)-1,3,5-triazine (simazine) and 2-azido-4-(i-propylamino)-6-methylthio-1,3,5-triazine (aziprotryne);

K. 1-alkoxy-1-alkyl-3-phenylurea herbicides such as 3-(3,4-dichlorophenyl)-1-methoxy-1-methylurea (linuron), 3-(4-chlorophenyl)-1-methoxy-1-methylurea (monolinuron) and 3-(4-bromo-4-chlorophenyl)-1-methoxy-1-methylurea (chlorobromuron).

L. thiolcarbamate herbicides such as S-propyl dipropylthiocarbamate (vernolate);

M. 1,2,4-triazin-5-one herbicides such as 4-amino-4,5-dihydro-3-methyl-6-phenyl-1,2,4-triazine-5-one (metamitron) and 4-amino-6-t-butyl-4,5-dihydro-3-methylthio-1,3,4-triazin-5-one (metribuzin);

N. benzoic acid herbicides such as 2,3,6-trichlorobenzoic acid (2,3,6-TBA), 3,6-dichloro-2-methoxybenzoic acid (dicamba) and 3-amino-2,5-dichlorobenzoic acid (chloramben);

O. anilide herbicides such as N-butoxymethyl-N-chloroacetyl 2',6'-diethylacetanilide (butachlor), the corresponding N-methoxy compound (alachlor), the corresponding N-i-propyl compound (propachlor) and 3',4'-dichloropropionanilide (propanil);

P. dihalobenzonitrile herbicides such as 2,6-dichlorobenzonitrile (dichlobenil), 3,5-dibromo-4-hydroxybenzonitrile (bromoxynil) and 3,5-diiodo-4-hydroxybenzonitrile (ioxynil).

Q. haloalkanoic herbicides such as 2,2-dichloropropionic acid (dalapon), trichloroacetic acid (TCA) and salts thereof;

R. diphenylether herbicides such as 4-nitrophenyl 2-nitro-4-trifluoromethylphenyl ether (fluorodifen), methyl 5-(2,4-dichlorophenoxy)-2-nitrobenzoate (bifenox), 2-nitro-5-(2-chloro-4-trifluoromethylphenoxy)benzoic acid, and 2-chloro-4-trifluoromethylphenyl 3-ethoxy-4-nitrophenyl ether.

S. miscellaneous herbicides including N,N-dimethyldiphenylacetamide (diphenamid), N-(1-naphthyl)phthalamic acid (naptalam) and 3-amino-1,2,4-triazole.

T. bipyridylium herbicides such as those in which the active entity is the 1,1'-dimethyl-4,4'-dipyridylium ion (paraquat) and those in which the active entity is the 1,1'-ethylene-2,2'dipyridylium ion (diquat).

U. Aryloxyphenoxypropionic acids and their derivatives (salts, esters, amides, and the like). Examples of such acids are:
2-[4-(3-chloro-5-trifluoromethylpyridyl-2-oxy)phenoxy]propionic acid.
2-[4-(5-trifluoromethylpyridyl-2-oxy)phenoxy]propionic acid.
2-[4-(4-trifluoromethylphenoxy)phenoxy]propionic acid.
2-[4-(2,4-dichlorophenoxy)phenoxy]propionic acid.
2-[4-(5- or 6-chlorobenzoxazolyl-2-oxy)phenoxy]propionic acid.
4-methyl-4-(4-trifluoromethylphenoxy)phenoxybut-2-enoic acid.

The acid chlorides (III) required as intermediates for preparing the compounds of the invention by the process of Scheme A may, as mentioned above, be prepared from the known carboxylic acids (II) by heating the latter under reflux in an excess of thionyl chloride and removing the excess of thionyl chloride when the reaction is complete. Reacting the acid chloride (II, X = $NO_2$) so prepared with excess of ammonia gave 5-(2-chloro-6-fluoro-4-trifluoromethylphenoxy)-2-nitrobenzamide as a white solid with a melting point of 176—177°C.

The invention is illustrated by the following Examples in which, unless otherwise stated, all parts are by weight, and all temperatures in degrees centigrade.

### Example 1

This Example illustrates the preparation of 1-methyl-1-ethyoxycarbonyl-methyl-5-(2-chloro-6-fluoro-4-trifluoromethylphenoxy)-2-nitrobenzoate (Compound No. 27 in Table 1).

5-(2-Chloro-6-fluoro-4-trifluoromethylphenoxy)-2-nitro-benzoic acid (1.03 g) was dissolved in dimethylsulphoxide (15 ml) and dry potassium carbonate (1 g) was added. The mixture was thoroughly stirred and a solution of ethyl-2-bromopropionate (0.49 g) in dimethylsulphoxide (5 ml) was added dropwise. The reaction mixture was stirred at room temperature for 3 hours and then left to stand

overnight. Dilute hydrochloric acid and water were added, and the product was extracted with ethyl acetate. The extract was washed, dried and concentrated to an oil. The product was purified by preparation thin layer chromatography and the structure (Compound No. 27 of Table 1) confirmed by elemental analysis and infra red and nmr spectroscopy.

Example 2

This Example illustrates the preparation of 2-chloroethyl-5-(2-chloro-6-fluoro-4-trifluoromethylphenoxy)-2-nitrobenzoate (Compound No. 1 of Table 1).

5-(2-Chloro-6-fluoro-4-trifluoromethylphenoxy)-2-nitro-benzoic acid was treated with thionyl chloride (20 ml), stirred and heated under reflux for 2 hours. The reaction mixture was evaporated under partial vacuum at 50°C to give a yellow oil. Toluene (20 ml) was added and then evaporated under partial vacuum to remove final traces of thionyl chloride from the acid chloride product.

The acid chloride product was dissolved in toluene (20 ml) and triethylamine (0.4 ml) was added dropwise followed by 2-chloroethanol (0.25 g). The cloudy mixture was stirred and gently heated under reflux until the reaction was complete (3 hours). The toluene was evaporated under partial vacuum and the residue was triturated with ether. The solid residue (the hydrochloric acid salt of triethylamine) was filtered off and the filtrate applied to the six 2 mm preparative thin layer chromatography plates (silica). The plates were developed in ether:hexane:acetic acid (60:40:5) and the faster major band removed. The product was extracted with ethyl acetate and evaporated under partial vacuum to give a yellow oil (0.9 g). The structure of the product (No. 1 of Table 1) was confirmed by elemental analysis and infra red and nmr spectroscopy.

Example 3

This Example illustrates the preparation of 2-nitrophenyl 5-(2-chloro-6-fluoro-4-trifluoromethylphenoxy)-2-nitrobenzoate (Compound No. 31 of Table 1).

5-(2-Chloro-6-fluoro-4-trifluoromethylphenoxy)-2-nitro-benzoic acid (1.03 g) was treated wtih thionyl chloride (20 ml) to form the acid chloride as described in Example 2. The resultant acid chloride product was dissolved in toluene (20 ml) and 2-nitrophenyl (0.38 g) added followed by triethylamine (0 4 ml). The mixture was stirred and heated under reflux until the reaction was complete (3-hours). The reation mixture was evaporated under partial vacuum and dilute hydrochloric acid was added followed by water. The product was extracted with ethyl acetate and the extract was washed with sodium hydroxide, brine and water and was finally dried over magnesium sulphate. The dry extract was concentrated by evaporation under partial vacuum and gave an oily residue which solidified on standing. The product was recrystalised from methanol to give 0.89 g of an off-white solid having a melting point of 105—106°C. The structure (Compound No. 31 of Table 1) was confirmed by elemental analysis, infra red and nmr spectroscopy and mass spectrometry.

Example 4

This Example illustrates the preparation of 0-[5-(2-chloro-6-fluoro-4-trifluorommethylphenoxy)-2-nitrobenzoyl]oxyimino-1-methylethylidene (Compound No. 37 of Table 1).

5-(2-Chloro-6-fluoro-4-trifluoromethylphenoxy)-2-nitro-benzoic acid (1.03 g) was treated with thionyl chloride (15 ml) to form the acid chloride as described in Example 2. The resultant acid chloride product was dissolved in toluene (20 ml) and acetoxime (0.2 g) was added followed by triethylamine (0.5 ml). The mixture was stirred and heated under reflux until the reaction was complete (3 hours). The reaction mixture was evaporated under partial vacuum and dilute hydrochloric acid was added followed by water. The product was extracted with ethyl acetate and the extract was washed, dried and concentrated to give a brown semi-solid residue. The residue was recrystallised from methanol to give 0.89 g of an off-white solid having a melting point of 105—106°C. The solid was purified by preparative thin layer chromatography (eluent ether: hexane: acetic acid—60: 40: 2) and the yellow residue following concentration of the extract was triturated with petrol (60—80) to give an off-white solid (0.62 g) having a melting point of 128—129°C. The structure (Compound No. 37 of Table 1) was confirmed by elemental analysis, and infra red and nmr spectroscopy.

Example 5

This Example illustrates the preparation of tetrahydrofurfuryl 5-(2-chloro-6-fluoro-4-trifluoromethylphenoxy)-2-nitrobenzoate (Compound No. 53 of Table 1).

5-(2-Chloro-6-fluoro-4-trifluoromethylphenoxy)-2-nitro-benzoic acid (1.03 g) was treated with thionyl chloride to form the acid chloride as described in Example 2. The resultant acid chloride product was dissolved in toluene (20 ml) and tetrahydrofurfuryl alcohol (0.3 g) dissolved in approximately 5 ml of toluene was added followed by triethylamine (0.5 ml). The mixture was stirred and heated under reflux until the reaction was complete (2 hours). The reaction mixture was evaporated under partial vacuum and dilute hydrochloric acid was added followed by water. The product was extracted with ethyl acetate and the extract was washed, dried and concentrated to give an oil. The oil was purified by preparative thin layer chromatography (eluent—ether: hexane: acetic acid—60: 40: 2) and a viscous orange-brown oil (0.58 g) was obtained. The structure (Compound No. 53 of Table 1) was confirmed by elemental analysis and infra red and nmr spectroscopy.

Example 6

This Example illustrates the preparation of 1-methylenepyrazole 5-(2-chloro-6-fluoro-4-trifluoromethylphenoxy)-2-nitrobenzoate (Compound No. 5 of Table 1).

5-(2-chloro-6-fluoro-4-trifluoromethylphenoxy)-2-nitro-benzoic acid (1.1 g) was treated with thionyl chloride to form the acid chloride as described in Example 2. The resultant acid chloride product was dissolved in toluene (20 ml) and triethylamine (0.5 ml) was added followed by 1-(hydroxymethyl)-pyrazole (0.5 g). The mixture was stirred and heated under reflux until the reaction was complete (4 hours). After cooling, the reaction mixture was filtered to remove a white solid (the hydrochloric acid salt of triethylamine), and the filtrate was evaporated to give a yellow oil. The oil was purified by preparative thin layer chromatography (eluent—ether: hexane: acetic acid—60: 40: 5) and the major band was removed with ethyl acetate. After evaporation, the resultant yellow oil was transferred to a vial in ether and evaporated to give a 0.8 g of a white solid mp. 88—89°C. The structure (Compound No. 5 of Table 1) was confirmed by elemental analysis, and infra red and nmr spectroscopy.

Example 7

This Example illustrates the preparation of N-methyl-5-(2-chloro-6-fluoro-4-trifluoromethylphenoxy)-2-nitrobenzamide (Compound No. 39 of Table 1).

5-(2-Chloro-6-fluoro-4-trifluoromethylphenoxy)-2-nitro- benzoic acid (1.03 g) was treated with thionyl chloride to form the acid chloride as described in Example 2. The resultant acid chloride product was dissolved in toluene, and the reaction mixture was stirred vigorously whilst methylamine gas was bubbled through the solution. Concentration gave an oily residue which was partitioned between water and ethyl acetate. The ethyl acetate extract was washed with brine dried over magnesium sulphate and concentrated to give a yellow sticky residue. The residue was purified by preparative thin layer chromatography (eluent—chloroform: acetone: acetic acid—90: 10: 5). The ethyl acetate extract of the major band concentrated to give a solid residue, which was triturated with ether/petroleum to give a white soil (0.73 g) having a melting point of 152—153°C. The structure (Compound No. 39 of Table 1) was confirmed by elemental analysis and nmr spectroscopy.

Example 8

This Example illustrates a preparation of N-ethoxycarbonylmethyl-5-(2-chloro-6-fluoro-4-trifluoromethylphenoxy)-2-nitrobenzamide (Compound No. 55 of Table 1). A solution of 5-(2-chloro-6-fluoro-4-trifluoromethylphenoxy)-2-nitrobenzoyl chloride (2.34 g) in carbon tetrachloride (15 ml) was added to a solution of glycine (0.43 g) in aqueous sodium hydroxide solution (10 ml of a solution containing 10 g per 100 ml of sodium hydroxide), followed by Triton B (3 drops) (Triton B is a Trade Mark for a solution of benzyltrimethyl ammonium hydroxide). The mixture was stirred at room temperature for four hours and then allowed to stand overnight. Ice was then added and the mixture acidified to Congo Red by addition of cncentrated hydrochloric acid. The aqueous layer was separated and extracted with ethyl acetate (3 × 75 ml). The extract was washed with water, (2 × 100 ml) dried ($Na_2SO_4$) and evaporated under reduced pressure to give a pale yellow solid (2.31 g) identified as N-carboxymethyl-5-(2-chloro-6-fluoro-4-trifluoromethylphenoxy)-2-nitrobenzamide. This was dissolved in ethanol (150 ml) and hydrogen chloride gas passed through the solution for 1 hour. The mixture was then heated under reflux for 3.5 hours, allowed to stand overnight, re-heated to reflux for a further 1.5 hours, and then allowed to stand for two days. The mixture was then evaporated under reduced pressure to give a yellow solid. This was dissolved in ethyl acetate (100 ml), and the solution washed with saturated sodium bicarbonate solution (3 × 70 ml) and water (100 ml), dried ($Na_2SO_4$) and evaporated to give a white solid (1.4 g).

This was then chromatographed on glass plates coated with a thin layer (2 mm) of silica gel, using a mixture of 3 volumes of toluene with 1 volume of ethyl acetate as the solvent. The fastest-running band was identified as ethyl 5-(2-chloro-6-fluoro-4-trifluoromethylphenoxy)-2-nitro benzoate. This was followed by a light blue fluorescent band and then by the required product. This was obtained as a white solid (0.9 g) with a melting point of 140—141°C.

Example 9

This Example illustrates the preparation of N-methoxy-[5-(2-choro-6-fluoro-4-trifluoromethylphenoxy)-2-nitrobenzamide] (Compound No. 57 of Table 1).

5-(2-Chloro-6-fluoro-4-trifluoromethylphenoxy)-2-nitro-benzoic acid (1.03 g) was treated with thionyl chloride to form the acid chloride as described in Example 2. The resultant acid chloride product was dissolved in toluene and added dropwise to a stirred solution of methyloxyamine hydrochloride (0.22 g) and triethylamine (0.5 ml) in toluene (20 ml). The reaction mixture was stirred at room temperature for 2 hours and left to stand at room temperature overnight. Concentration gave an oily residue which was partitioned between water and ethyl acetate. The ethyl acetate extract was washed, dried and concentrated to an oil which was purified by preparative thin layer chromatography (eluent—chloroform: acetone: acetic acid—90: 10: 5). The ethyl acetate extract of the major band concentrated to give a residue, which was triturated with ether/petroleum to give a white solid (0.21 g) having a melting point of 162—163°C. The structure (Compound No. 57 of Table 1) was confirmed by elemental analysis and nmr spectroscopy.

Example 10

This Example illustrates the preparation of 0-[5-(2-chloro-6-fluoro-4-trifluoromethylphenoxy)-2-nitrobenzoyl]-oxyimino-1-ethoxycarbonylethylidene (Compound No. 59 of Table 1).

5-(2-Chloro-6-fluoro-4-trifluoromethylphenoxy)-2-nitrobenzoic acid (1.03 g) was treated with thionyl chloride to form the acid chloride as described in Example 2. The resultant acid chloride product was dissolved in toluene (15 ml) and ethyl acetoacetate oxime (0.4 g) was added dropwise followed by triethylamine (0.5 ml). The mixture was stirred and gently heated under reflux at 110°C to 120°C for 3 hours and then left to stand overnight. The reaction mixture was concentrated and was partitioned between water and ethyl acetate. The ethyl acetate extract was washed, dried and concentrated to give an oil. The oil was purified by preparative thin layer chromatography (eluent ethyl acetate: hexane: acetic acid 60 : 40 : 2) and the product was triturated with ether/petrol to give an offwhite solid (0.48 g) having a melting point of 106—108°C. The structure (compound No. 59 of Table 1) was confirmed by elemental analysis, nmr spectroscopy and mass spectroscopy.

Example 11

This Example illustrated the preparation of 1-methyl-1-ethoxycarbonyl-methyl 2-chloro-5-(2-chloro-6-fluoro-4-trifluoromethylphenoxy)-benzoate (Compound No. 28 of Table 1).

The procedure of Example 1 was repeated except that instead of 5-(2-chloro-6-fluoro-4-trifluoromethylphenoxy)-2-nitrobenzoic acid as starting material there was used 2-chloro-5-(2-chloro-6-fluoro-4-trifluoromethylphenoxy)benzoic acid (1.11 g). The product was an oil whose structure (Compound No. 28 of Table 1) was confirmed by elemental analysis and infra red and nmr spectroscopy.

Example 12

This Example illustrated the preparation of 0-[2-chloro-5-(2-chloro-6-fluoro-4-trifluoromethylphenoxy)-benzoyl)-oxyimino-1-methylethylidene (compound 38 of Table 1).

The precedure of Example 4 was repeated except that instead of 5-(2-chloro-6-fluoro-4-trifluoromethylphenoxy)-2-nitrobenzoic acid as starting material there was used 2-chloro-5-(2-chloro-6-fluoro-4-trifluorohypherate and take over methylphenoxy)benzoic acid (1.11 g). The product was a viscous yellow oil which crystallised on standing to a solid mp 72—75°C, and whose structure (Compound No. 38 of Table 1) was confirmed by elemental analysis and infra red and nmr spectroscopy.

Example 13

The Example illustrates the preparation of tetrahydrofurfuryl 2-chloro-5-(2-chloro-6-fluoro-4-trifluoromethylphenoxy)benzoate (Compound No. 54 of Table 1).

The procedure of Example 5 was repeated except that instead of 5-(2-chloro-6-fluoro-4-trifluoromethylphenoxy)-2-nitrobenzoic acid as starting material there was used 2-chloro-5-(2-chloro-6-fluoro-4-trifluoromethylphenoxy)benzoic acid (1.11 g). The product was an oil whose structure (compound No. 54 of Table 1) was confirmed by elemental analysis and infra red and nmr spectroscopy.

Example 14

This example illustrated the preparation of 1-methylenepyrazole 2-chloro-5-(2-chloro-6-fluoro-4-trifluoromethylphenoxy)-benzoate (Compound No. 6 of Table 1).

The procedure of Example 6 was repeated except that instead of 5-(2-chloro-6-fluoro-4-trifluoromethylphenoxy)-2-nitrobenzoic acid as starting material there was used 2-chloro-5-(2-chloro-6-fluoro-4-trifluoromethylphenoxy)-benzoic acid (0.75 g) and the quantity of 1-hydroxymethylpyrazole was 0.21 g. The product was a white solid having a melting point of 97—99°C, whose analysis, nmr spectroscopy and mass spectroscopy.

Example 15

This Example illustrated the preparation of N-methyl-2-chloro-5-(2-chloro-6-fluoro-4-trifluoromethylphenoxy)-benzamide (Compound No. 40 of Table 1).

The procedure of Example 7 was repeated except that instead of 5-(2-chloro-6-fluoro-4-trifluoromethylphenoxy)-2-nitrobenzoic acid as starting material there was used 2-chloro-5-(2-chloro-6-fluoro-4-trifluoromethylphenoxy)benzoic acid (1.11 g). The product was a solid having a melting point of 132—133.5°C, whose structure (Compound No. 40 of Table 1) was confirmed by elemental analysis, nmr spectroscopy and mass spectrometry.

Example 16

This Example illustrates a preparation of N-ethoxycarbonyl methyl-2-chloro-5(2-chloro-6-fluoro-4-trifluoromethylphenoxy)benzamide (Compound No. 56 of Table 1).

2-Chloro-5-(2-chloro-6-fluoro-4-trifluoromethylphenoxy)benzoic acid (1.5 g) was heated under reflux in excess of thionyl chloride. The excess of thionyl chloride was removed under reduced pressure. The remaining oil was taken up in dry ether and slowly added at room temperature with vigorous stirring to a freshly prepared solution of glycine ethyl ester. (This solution was prepared by treating glycine ethyl ester hydrochloride (0.56 g) with an equimolar amount of triethylamine in dichloromethane. The dichloromethane was removed under reduced pressure and the residue stirred with ether and filtered to remove triethylamine hydrochloride). The mixture was stirred for 30 minutes and the ether removed under reduced pressure. The remaining oil was shaken with ethyl acetate and water. The ethyl acetate layer was separated, washed twice with water, dried ($MgSO_4$) and evaporated under reduced pressure. The oil was taken up in boiling ether, and hexane added, and the solution cooled. The solid which separated was recrystallised from a mixture of chloroform and hexane to give N-ethoxycarbonylmethyl-2-chloro-5-(2-chloro-6-fluoro-4-trifluoromethylphenoxy) benzamide, 0.47 g) with a melting point of 94°C.

Example 17

This Example illustrated the preparation of 0-[2-chloro-5-(2-chloro-6-fluoro-4-trifluoromethylphenoxy)benzoyl]-oxyimino-1-ethoxycarbonylethylidene (Compound 60 of Table 1).

The procedure of Example 10 was repeated except that instead of 5-(2-chloro-6-fluoro-4-trifluoromethylphenoxy)-2-nitrobenzoic acid as starting material there was used 2-chloro-5-(2-chloro-6-fluoro-4-trifluoromethylphenoxy)-benzoic acid (1.11 g). The product was a solid having a melting point of 80—82°C, whose structure (Compound No. 60 of Table 1) was confirmed by elemental analaysis, nmr spectroscopy and mass spectrometry.

Example 18

This Example illustrated the preparation of N-(1-methyl-1-ethoxylcarbonyl-methyl)-2-chloro-5-(2-chloro-6-fluoro-4-trifluoromethylphenoxy)-benzamide (Compound No. 62 of Table 1).

2-Chloro-5-(2-chloro-6-fluoro-4-trifluoromethylphenoxy)benzoic acid (1.03 g) was treated with thionyl chloride (15 ml) to form the acid chloride as described previously. The resultant acid chloride product was dissolved in toluene (5 ml) and added dropwise to a stirred mixture of D.L-alamine ethyl ester (0.35 g) and triethylamine (0.5 ml) in toluene (15 ml). The mixture was stirred at room temperature for 2 hours and left to stand overnight. The reaction mixture was concentrated and partitioned between water and ethyl acetate. The organic extract was washed, dried and concentrated to gived an oil. The oil was purified by reparative thin layer chromatography (eluent—ether: hexane: acetic acid—60 : 40 : 2) and a very viscous oil was obtained. The structure (Compound No. 62 of Table 1) was confirmed by elemental analysis, nmr spectroscopy and gas chromatography/mass spectrometry.

Example 19

This Example illustrates a preparation of N-methoxycarbonyl-5-(2-chloro-6-fluoro-4-trifluoromethylphenoxy)-2-nitrobenzamide (Compound No. 63 of Table 1).

The known compound 3-(2-chloro-6-fluoro-trifluoromethylphenoxy)benzoic acid was heated under reflux in excess of thionyl chloride for 3 hours. The excess of thionyl chloride was removed under reduced pressure. The oil which remained (3-(2-chloro-6-fluoro-4-trifluoromethylphenoxy) benzoyl chloride) was treated with excess of aqueous ammonia to give 3-(2-chloro-6-fluoro-4-trifluoromethylphenoxy)benzamide. A sample of this material (1.67 g) was heated under reflux with oxalyl chloride (4 ml) in 1,2- and oxalyl chloride removed under reduced pressure. Methanol (100 ml) was added to the brown oil which remained, and the solution heated under reflux for 1 hour and then left overnight at room temperature. The excess of methanol was removed under reduced pressure and the remaining solid recrystallised from a mixture of dichloromethane and petroleum ether (b.p. 40—60°), using decolourising charcoal. The N-methoxy-carbonyl-3-(2-chloro-6-fluoro-4-trifluoro-methylphenoxy) benzamide so obtained (1.65 g) had a melting point of 128—129°C.

The substituted benzamide derivative so prepared (1.18 g) was added in small portions to a mixture of 1,2-dichloroethane (10 ml) and concentrated sulphuric acid (10 ml) stirred vigorously at 0°C. Potassium nitrate (0.33 g) was then added in small portions over a period of 15 minutes, keeping the temperature at 0°C. The mixture was stirred for a further 30 minutes at 0°C and then allowed to warm to room temperature. The mixture was poured into ice and water (50 ml) and extracted with ether. The extract was washed with saturated brine, dried, and filtered. Petroleum ether (b.p. 40—60°) was added and the product separated as a colourless powder, (0.8 g) with a melting point of 144—146°C. The analytical and spectral data were consistent with the identification of the compound as N-methoxycarbonyl-5-(2-chloro-6-fluoro-4-trifluoromethylphenoxy)-2-nitrobenzamide.

Examples 20 to 29

These Examples illustrate the herbicidal properties of the compounds of Examples 1 to 10 respectively. The compounds were each submitted to herbicide tests as described below.

The compound was formulated for test by mixing an appropriate amount of it with 5 ml of an emulsion prepared by diluting 160 ml of a solution containing 21.8 g per litre of Span 80 and 78.2 g per litre of Tween 20 in methylcyclohexanone to 500 ml with water. Span 80 is a Trade Mark for a surface-active agent comprising sorbitan monolaurate. Tween 20 is a Trade Mark for a surface-active agent comprising a condensate of 20 molar proportions of ethylene oxide with sorbitan monolaurate. The mixture of the compound and the emulsion was then shaken with glass beads and diluted to 40 ml with water. The spray composition so prepared was sprayed onto young pot plants (post-emergence test) of the species named in the Table below, at a rate equivalent to 1000 litres per hectare. Damage to plants was assessed 14 days

after spraying by comparison with untreated plants, on a scale of 0 to 5 where 0 is 0 to 20% damage and 5 is complete kill. In the table of results, a dash (−) means that no test was made.

A test was also carried out to detect pre-emergence herbicidal activity. Seeds of the test species were placed on the surface of fibre trays of soil and were sprayed with the compositions at the rate of 1000 litres per hectare. The seeds were then covered with further soil. Three weeks after spraying, the seedlings in the sprayed fibre trays were compared with the seedlings in unsprayed control trays, the damage being assessed on the same scale of 0 to 5.

The results of the tests are given in the Table II below.

TABLE II

| Compound of Example | Rate of application (kg/ha) | Pre or Post Emergence | Test Plants | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Ga | Xa | Ab | Co | Av | Dg | Al | St | Ec | Sh | Ag | Cn |
| 1 | 0.2 | Pre | 3 | 3 | 0 | 0 | 0 | 3 | 4 | 5 | 3 | 4 | 4 | 5 | — | 3 | 0 | 0 | 1 | 2 | 0 | 0 | 0 | 4 | 4 | 1 |
| | 0.1 | Post | 3 | 3 | 5 | 2 | 3 | 1 | 4 | 4 | 5 | 5 | 3 | 4 | 5 | 5 | 4 | 4 | 1 | 2 | 2 | 3 | 2 | 1 | 0 | 3 |
| 2 | 0.2 | Pre | 3 | 3 | 0 | — | 0 | 3 | 4 | 5 | 3 | 5 | 3 | 4 | — | 3 | 1 | 1 | 3 | 2 | 1 | 2 | 0 | 4 | 4 | 2 |
| | 0.5 | Post | 3 | 5 | 5 | 3 | 3 | 2 | 4 | 5 | 5 | 5 | 2 | 4 | 5 | 4 | 5 | 2 | 4 | 3 | 4 | 3 | 2 | 2 | 1 | 4 |
| 3 | 1.0 | Pre | 4 | 4 | 0 | — | 2 | 4 | 4 | 5 | 3 | 5 | 2 | 3 | — | 4 | 3 | 3 | 1 | 2 | 1 | 4 | 0 | 4 | 4 | 2 |
| | 0.5 | Post | 2 | 3 | 4 | 2 | 3 | 1 | 4 | 4 | 5 | 3 | 1 | 4 | 4 | 3 | 3 | 2 | 1 | 2 | 0 | 2 | 1 | 2 | 0 | 0 |
| 4 | 0.2 | Pre | 5 | 5 | 0 | 0 | 3 | 4 | 4 | 5 | 3 | 5 | 1 | 3 | — | 5 | 2 | 0 | 1 | 2 | 1 | 3 | 5 | 4 | 3 | 1 |
| | 0.5 | Post | 3 | 5 | 5 | 2 | 4 | 4 | 4 | 5 | 5 | 5 | 5 | — | 5 | 4 | 4 | 4 | 5 | 4 | 4 | 3 | 5 | 4 | 3 | 2 |
| 5 | 1,0 | Pre | 4 | 5 | 1 | 0 | 4 | 4 | 5 | 5 | 4 | 5 | 5 | 5 | — | 5 | 5 | 1 | 4 | 5 | 4 | 4 | 5 | 5 | 5 | 3 |
| | 0.1 | Post | 3 | 3 | 4 | 3 | 3 | 2 | 3 | 5 | 4 | 5 | 4 | — | 5 | 4 | 5 | 3 | 3 | 3 | 2 | 2 | 1 | 3 | 1 | 2 |
| 6 | 0.2 | Pre | 5 | 4 | 0 | 1 | 2 | 4 | 4 | 5 | 3 | 5 | 3 | 4 | — | 5 | 1 | 0 | 2 | 2 | 1 | 3 | 3 | 4 | 4 | 1 |
| | 0.1 | Post | 4 | 4 | 4 | 2 | 3 | 3 | 3 | 5 | 5 | 5 | 5 | 5 | — | 5 | 4 | 5 | 3 | 3 | 3 | 3 | 3 | 3 | 1 | 1 |
| 7 | 0.05 | Pre | 4 | 4 | 1 | 2 | 3 | 4 | 4 | 5 | 2 | 5 | 3 | 5 | — | 1 | 0 | 5 | 2 | 1 | 2 | 2 | 4 | 2 | 3 | 3 |
| | 0.1 | Post | 4 | 3 | 4 | 3 | 2 | 3 | 2 | 3 | 4 | 4 | 3 | 4 | 5 | 3 | 4 | 3 | 1 | 2 | 2 | 3 | 2 | 3 | 1 | 1 |
| 8 | 1.0 | Pre | 5 | 5 | 3 | 1 | 4 | 4 | 5 | 5 | 5 | 5 | 4 | 5 | — | 5 | 5 | 4 | 4 | 1 | 4 | 5 | 5 | 4 | 4 | 4 |
| | 0.5 | Post | 3 | 5 | 5 | 4 | 4 | 3 | 2 | 5 | 5 | 4 | 4 | 5 | 5 | 4 | 5 | 4 | 2 | 3 | 3 | 3 | 4 | 4 | 1 | 2 |
| 9 | 0.2 | Pre | 5 | 5 | 1 | 1 | 4 | 4 | 4 | 5 | 3 | 5 | 5 | 5 | — | 2 | 3 | 3 | 4 | 1 | 3 | 3 | 4 | 3 | 4 | 2 |
| | 0.5 | Post | 5 | 4 | 5 | 3 | 3 | 2 | 3 | 4 | 5 | 5 | 5 | 4 | 5 | 4 | 4 | 3 | 1 | 1 | 2 | 3 | 2 | 3 | 2 | 1 |
| 10 | 0.2 | Pre | 5 | 5 | 1 | 1 | 2 | 4 | 4 | 5 | 4 | 5 | 5 | — | — | 3 | 5 | — | 3 | 4 | 3 | 5 | 4 | 5 | 4 | — |
| | 0.2 | Post | 5 | 5 | 4 | 4 | 4 | 4 | 5 | 5 | 5 | 5 | 5 | 4 | — | 5 | 5 | 4 | 4 | 5 | 3 | — | 5 | 5 | 2 | 3 |

Names of test plants in Table II

Sb Sugar beet
Rp Rape
Ct Cotton
Sy Soya Bean
Mz Maize
Ww Winter wheat
Rc Rice
Sn *Senecio vulgaris*
Ip *Ipomoea purpurea*
Am *Amaranthus retroflexus*
Pi *Polygonum aviculare*
Ca *Chenopoldium album*
Ga *Galium aparine*
Xa *Xanthium spinosum*
Ab *Abutilon theophrastii*
Ot/Av Oats (Cultivated in pre-emergence test and *Avena fatua* (wild oats) in post-emergence test)
Dg *Digitaria sanguinalis*
Al *Alopecurus myosuriodes*
St *Setaria viridis*
Ec *Echinochloa crus-galli*
Sh *Sorghum halepense*
Ag *Agropyron repens*
Cn *Cyperus rotundus*

**Claims**

1. Diphenyl ether compounds of the formula (I):

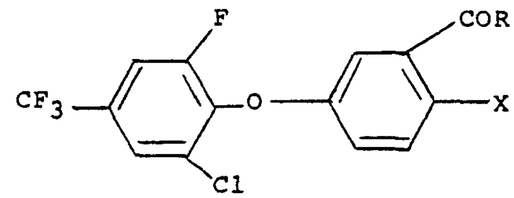

(I)

wherein X is a nitro group or a chlorine atom and R is:

(a) a group $ZR^3$ wherein Z is oxygen or sulphur, and $R^3$ is an aliphatic or alicyclic radical of up to 6 carbon atoms bearing one or more of the following substituents: halogen; nitro; hydroxy; $CF_3$; alkoxy containing 1 to 4 carbon atoms; alkylthio, alkylsulphinyl, or alkylsulphonyl each having from 1 to 4 carbon atoms; phenyl; heterocyclyl containing 5 or 6 ring atoms; a group $—NR^1R^2$ as defined below; cyano; carboxy or a salt thereof; alkoxycarbonyl having from 2 to 5 carbon atoms; a group $—CONR^1R^2$ wherein $—NR^1R^2$ is as defined below; formyl; alkanoyl having for example 2 to 5 carbon atoms; or a 1-pyrazolyl, 1-imidazolyl, 1-(1,2,4-triazolyl) or 2-(1,2,3-triazolyl) group optionally bearing one or more halogen, methyl, methoxy, or methylthio substituents.

(b) a group $—NR^1R^2$ wherein $R^1$ is hydrogen, an optionally substituted aliphatic radical of 1 to 6 carbon atoms or an optionally substituted alicyclic radical of 3 to 6 carbon atoms or an optionally substituted phenyl radical, and $R^2$ is defined as for $R^1$ but may additionally represent hydroxy; alkoxy of 1 to 4 carbon atoms; alkoxy of 1 to 4 carbon atoms substituted by phenyl or by carboxy or a salt, ester or amide thereof; or alkoxy of 1 to 4 carbon atoms substituted by amino or mono- or di-dialkylamino wherein the alkyl groups have from 1 to 4 carbon atoms; or wherein the group $—NR^1R^2$ is constituted by an optionally methyl-substituted pyrrolidine, piperidine, or morpholine ring, or by a succinimido, or phthalimido radical or by a group of the formula:

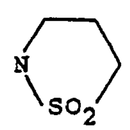

or

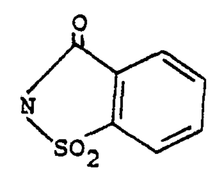

or

(c) a group $—NS^1S^2$ wherein $S^1$ is hydrogen, an alkyl group (eg. $C_1$ to $C_4$ alkyl), an agriculturally acceptable cation, or chlorine, bromine, or iodine; and $S^2$ is a group

$$—\underset{\underset{O}{\|}}{C}OS^3$$

wherein $S^3$ is an optionally substituted aliphatic or alicyclic radical (eg. having from 1 to 6 carbon atoms), or an optionally substituted phenyl radical; or $S^2$ is a group

$$—(\overset{S^9\ \ S^4}{C})_n—S^5$$

wherein $S^4$ and $S^9$ are independently hydrogen or a $C_1$ to $C_4$ alkyl group or phenyl group and n is 1, 2, or 3, and $S^5$ is a CN group, a group $—CONS^6S^7$ wherein each of $S^6$ and $S^7$ is hydrogen, an optionally substituted aliphatic or alicyclic radical (eg. having from 1 to 6 carbon atoms) or an optionally substituted phenyl radical; or $S^5$ is a group

$$—\underset{\underset{O}{\|}}{C}OS^8$$

wherein $S^8$ is hydrogen, an agriculturally acceptable cation, or an optionally substituted aliphatic or alicyclic radical (having for example from 1 to 6 carbon atoms) or an optionally substituted phenyl radical, or

(d) an optionally substituted phenoxy, phenylthio, or 2-sulpholanoxy group, or a group $—ON{=}CR^4R^5$ wherein $R^4$ is hydrogen, halogen, alkyl, mononuclear aryl, $(C_1—C_6)$alkoxy, $(C_1—C_6)$alkylthio or

dialkylamino; and $R^5$ is substituted or unsubstituted alkyl, trifluoromethyl, cyano, carboxy, substituted or unsubstituted alkoxy, alkoxycarbonyl, alkylcarbonyl, alkylthio, aminocarbonyl, mononuclear arylcarbonyl, mononuclear aryloxy or carboxy including agronomically acceptable amides, esters or salts of said carboxy; or $R^4$ and $R^5$ may be joined together to form a ring containing from 4 to 8 nuclear carbon atoms.

2. A compound as claimed in claim 1 wherein, in formula (I), X is a nitro group or a chlorine atom and R is (a) a group $ZR^3$ or (b) a group $NR^1R^2$ or (c) a phenoxy, phenylthio, 2-nitrophenoxy, or 2-sulpholanoxy group, or a group $—ON{=}C(CH_3)_2$.

3. A compound as claimed in claim 1 wherein, in formula (I), X is a nitro group or a chlorine atom and R is a group $—NS^1S^2$.

4. A compound as claimed in claim 1 wherein R is a group $ZR^3$ wherein Z is oxygen and $R^3$ is an alkyl radical of 1 to 6 carbon atoms substituted by one or more of the following:

a halogen atom; a nitro group; a carboxyl group or a salt or $C_{1-6}$ alkyl ester thereof; or an optionally chloro, methyl, or methoxy substituted 1-imidazolyl, 1-(1,2,4-triazolyl), or 1-pyrazolyl group.

5. A compound as claimed in claim 1 wherein R is a group $ZR^3$ which comprises a phenoxy or phenylthio group optionally substituted by nitro.

6. A compound as claimed in claim 1 wherein R is a group $NR^1R^2$ wherein $R^1$ is hydrogen or alkyl of 1 to 6 carbon atoms and $R^2$ is hydrogen, alkoxy of 1 to 6 carbon atoms, or alkoxy of 1 to 6 carbon atoms substituted by carboxy or a salt of $C_{1-6}$ alkyl ester thereof, or wherein the group $—NR^1R^2$ is constituted by an optionally methyl-substituted pyrrolidine, piperidine or morpholine ring, or by a succinimido or phthalimido radical, or by a group of the formula:

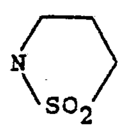

or

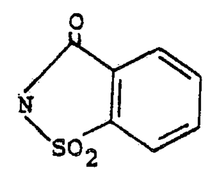

7. A compound as claimed in claim 3 wherein R is a group $—NS^1S^2$ wherein $S^1$ is hydrogen and $S^2$ is a group $—CHS^9S^5$ wherein $S^9$ is hydrogen or a $C_1$ to $C_4$ alkyl group and $S^5$ is a group $—CO.OS^8$ wherein $S^8$ is hydrogen or a cation or a $C_1$ or $C_6$ alkyl group.

8. A compound as claimed in claim 1 wherein R is a group $—ON{=}CR^4R^5$ wherein $R^4$ is $C_1$ to $C_4$ alkyl and $R^5$ is $C_1$ to $C_4$ alkyl optionally substituted by alkoxy ($C_1$ to $C_4$) carbonyl.

9. A process of inhibiting the growth of unwanted plants, which comprises applying to the plants, or to the locus thereof, a phytotoxic amount of a compound as claimed in any of the preceding claims.

10. A process of preparing a compound of the formula (I) defined in claim 1, which comprises reacting a compound of formula

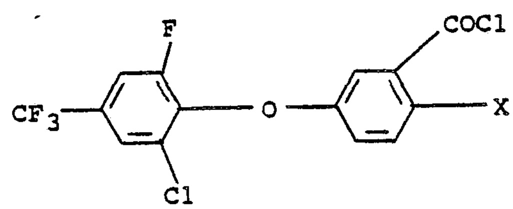

with a compound of formula RH in the presence of an acid acceptor and recovering the compound of formula (I).

11. A process of preparing a compound of formula (I) as defined in claim 1 which comprises reacting a compound of the formula:

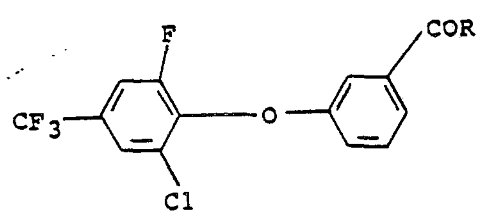

with a nitrating agent or halogenating agent and recovering the compound of formula (I).

12. Herbicidal compositions, comprising as an active ingredient a compound according to any of claims 1 to 7 in admixture with a carrier comprising a solid or liquid diluent.

**Patentansprüche**

1. Diphenylether-Verbindungen der Formel (I)

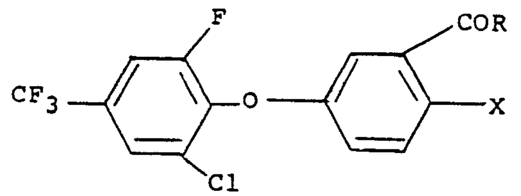

(I)

worin X für eine Nitro-Gruppe oder für ein Chlor-Atom steht und R für folgendes steht:

(a) ein Gruppe —$ZR^3$, worin Z Sauerstoff oder Schwefel bedeutet und $R^3$ ein aliphatisches oder alicyclisches Radikal mit bis zu 6 Kohlenstoffatomen bedeutet, das ein oder mehrere der folgenden Substituenten trägt: Halogen; Nitro; Hydroxy; —$CF_3$; Alkoxy mit 1 bis 4 Kohlenstoffatomen; Alkylthio, Alkylsulfinyl oder Alkylsulfonyl mit jeweils 1 bis 4 Kohlenstoffatomen; Phenyl; Heterocyclyl mit 5 oder 6 Ringatomen; eine Gruppe —$NR^1R^2$, wie sie unten definiert ist; Cyano; Carboxy oder ein Salz davon; Alkoxycarbonyl mit 2 bis 5 Kohlenstoffatomen; eine Gruppe —$CONR^1R^2$, worin —$NR^1R^2$ die unten angegebene Definition besitzt; Formyl; Alkanoyl mit beispielsweise 2 bis 5 Kohlenstoffatomen; oder eine 1-Pyrazolyl-, 1-Imidazolyl-, 1-(1,2,4-Triazolyl)- oder 2-(1,2,3-Triazolyl)-Gruppe, die gegebenenfalls ein oder mehrere Halogen-, Methyl-, Methoxy- oder Methylthio-Substituenten trägt;

(b) ein Gruppe —$NR^1R^2$, worin $R^1$ Wasserstoff, ein gegebenenfalls substituiertes aliphatisches Radikal mit 1 bis 6 Kohlenstoffatomen oder ein gegebenenfalls substituiertes alicyclisches Radikal mit 3 bis 6 Kohlenstoffatomen oder ein gegebenenfalls substituiertes Phenyl-Radikal bedeutet, und $R^2$ die gleiche Definition wie $R^1$ aufweist, aber noch zusätzlich folgendes bedeuten kann: Hydroxy; Alkoxy mit 1 bis 4 Kohlenstoffatomen; Alkoxy mit 1 bis 4 Kohlenstoffatomen, das durch Phenyl oder Carboxy oder ein Salz, einen Ester oder ein Amid davon substituiert ist; oder Alkoxy mit 1 bis 4 Kohlenstoffatomen, das durch Amino oder Mono- oder Dialkylamino, worin die Alkyl-Gruppen 1 bis 4 Kohlenstoffatome aufweisen, substituiert ist; oder worin die Gruppe —$NR^1R^2$ aus einem gegebenenfalls methylsubstituierten Pyrrolidin-, Piperidin- oder Morpholin-Ring oder aus einem Succinimido- oder Phthalimido-Radikal oder einer Gruppe der Formel

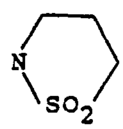

oder

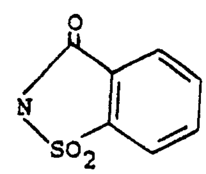

besteht;

(c) eine Gruppe —$NS^1S^2$, worin $S^1$ Wasserstoff, eine Alkyl- Gruppe (z.B. $C_{1-4}$-Alkyl), ein landwirtschaftlich zulässiges Kation oder Chlor, Brom oder Jod bedeutet und $S^2$ eine Gruppe

$$-\underset{\underset{O}{\|}}{C}OS^3$$

bedeutet, worin $S^3$ ein gegebenenfalls substituiertes aliphatisches oder alicyclisches Radikal (z.B. mit 1 bis 6 Kohlenstoffatomen) oder ein gegebenenfalls substituiertes Phenyl-Radikal darstellt; oder $S^2$ eine Gruppe

$$-(\overset{S^9\ \ S^4}{\overset{\diagdown\ \diagup}{C}})_n-S^5$$

bedeutet, worin $S^4$ und $S^9$ unabhängig Wasserstoff oder eine $C_{1-4}$-Alkyl-Gruppe oder eine Phenyl-Gruppe darstellen, n 1, 2 oder 3 darstellt und $S^5$ eine Gruppe —CN oder eine Gruppe —$CONS^6S^7$ darstellt, worin $S^6$ und $S^7$ jeweils Wasserstoff, ein gegebenenfalls substituiertes aliphatisches oder alicyclisches Radikal (z.B. mit 1 bis 6 Kohlenstoffatomen) oder ein gegebenenfalls substituiertes Phenyl-Radikal sind; oder $S^5$ eine Gruppe

$$-\underset{\underset{O}{\|}}{C}OS^8$$

darstellt, worin $S^8$ Wasserstoff, ein landwirtschaftlich zulässiges Kation oder ein gegebenenfalls

substituiertes aliphatisches oder alicyclisches Radikal (mit beispielsweise 1 bis 6 Kohlenstoffatomen) oder ein gegebenenfalls substituiertes Phenyl-Radikal ist; oder

(d) eine gegebenenfalls substituierte Phenoxy-, Phenylthio- oder 2-Sulfolanoxy-Gruppe oder eine Gruppe —ON=$CR^4R^5$, worin $R^4$ Wasserstoff, Halogen, Alkyl, einkerniges Aryl, $C_{1-6}$-Alkoxy, $C_{1-6}$-Alkylthio oder Dialkylamino bedeutet und $R^5$ substituiertes oder unsubstituiertes Alkyl, Trifluoromethyl, Cyano, Carboxy, substituiertes oder unsubstituiertes Alkoxy, Alkoxycarbonyl, Alkylcarbonyl, Alkylthio, Aminocarbonyl, einkerniges Arylcarbonyl, einkerniges Aryloxy oder Carboxy einschließlich landwirtschaftlich zulässiger Amide, Ester oder Salze von diesem Carboxy bedeutet; oder $R^4$ und $R^5$ miteinander verbunden sein können, so daß sie einen Ring mit 4 bis 8 Kernkohlenstoffatomen bilden.

2. Verbindung nach Anspruch 1, worin in der Formel (I) X für eine Nitro-Gruppe oder ein Chlor-Atom steht und R (a) für eine Gruppe —$ZR^3$ oder (b) ein Gruppe —$NR^1R^2$ oder (c) eine Phenoxy-, Phenylthio-, 2-Nitrophenoxy- oder 2-Sulfolanoxy-Gruppe oder eine Gruppe —ON=$C(CH_3)_2$ steht.

3. Verbindung nach Anspruch 1, worin in der Formel (I) X für eine Nitro-Gruppe oder ein Chlor-Atom steht und R für eine Gruppe —$NS^1S^2$ steht.

4. Verbindung nach Anspruch 1, worin R für eine Gruppe —$ZR^3$ steht, worin Z sauerstoff bedeutet und $R^3$ ein Alkyl-Radikal mit 1 bis 6 Kohlenstoffatomen bedeutet, das durch ein oder mehrere der folgenden Substituenten substituiert ist: ein Halogen-Atom; eine Nitro-Gruppe; eine Carboxyl-Gruppe oder ein Salz oder einen $C_{1-6}$-Alkyl- est davon; oder eine gegebenenfalls chloro-, methyl- oder methoxy-substituierte 1-Imidazolyl-, 1-(1,2,4-Triazolyl)- oder 1-Pyrazolyl-Gruppe.

5. Verbindung nach Anspruch 1, worin R für eine Gruppe —$ZR^3$ steht, welche eine Phenoxy- oder Phenylthio- Gruppe, die gegebenenfalls durch Nitro substituiert ist, umfaßt.

6. Verbindung nach Anspruch 1, worin R für eine Gruppe —$NR^1R^2$ steht, worin $R^1$ Wasserstoff oder Alkyl mit 1 bis 6 Kohlenstoffatomen bedeutet und $R^2$ Wasserstoff, Alkoxy mit 1 bis 6 Kohlenstoffatomen oder Alkoxy mit 1 bis 6 Kohlenstoffatomen, das durch Carboxy oder ein Salz oder einen $C_{1-6}$-Alkyl-Ester davon substituiert ist, bedeutet, oder worin die Gruppe —$NR^1R^2$ aus einem gegebenenfalls methyl substituierten Pyrrolidin-, Piperidin- oder Morpholin-Ring oder ein Succinimido- oder Phthalimido- Radikal oder eine Gruppe der Formel

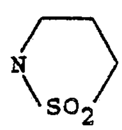

oder

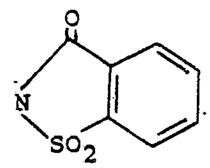

besteht.

7. Verbindung nach Anspruch 3, worin R für eine Gruppe —$NS^1S^2$ steht, worin $S^1$ Wasserstoff bedeutet und $S^2$ eine Gruppe —$CHS^9S^5$ bedeutet, worin $S^9$ Wasserstoff oder eine $C_{1-4}$-Alkyl-Gruppe darstellt und $S^5$ eine Gruppe —$CO.OS^8$ darstellt, worin $S^8$ Wasserstoff oder ein Kation oder eine $C_{1-6}$-Alkyl-Gruppe ist.

8. Verbindung nach Anspruch 1, worin R für eine Gruppe —ON=$CR^4R^5$ steht, worin $R^4$ $C_{1-4}$-Alkyl bedeutet und $R^5$ $C_{1-4}$-Alkyl, das gegebenenfalls durch $C_{1-4}$-Alkoxycarbonyl substituiert ist, bedeutet.

9. Verfahren zur Verhinderung des Wachstums von unerwünschten Pflanzen, bei welchem auf die Pflanzen oder auf den Standort der Pflanzen eine phytotoxische Menge einer Verbindung nach einem der vorhergehenden Ansprüche aufgebracht wird.

10. Verfahren zur Herstellung einer Verbindung der formel (I) von Anspruch 1, bei welchem eine Verbindung der Formel

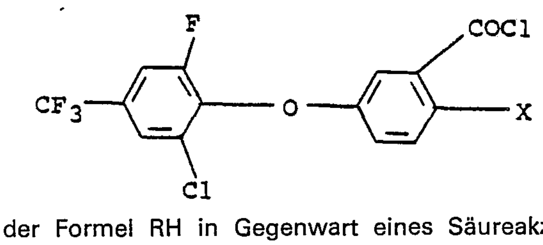

mit einer Verbindung der Formel RH in Gegenwart eines Säureakzeptors umgesetzt wird und die Verbindung der Formel (I) abgetrennt wird.

11. Verfahren zur Herstellung einer Verbindung der Formel (I) von Anspruch 1, bei welchem eine Verbindung der Formel

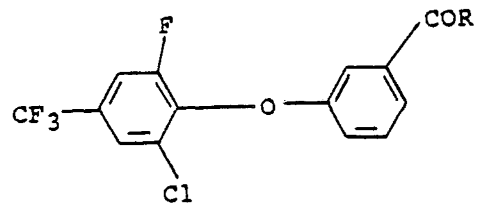

mit einem Nitrierungsmittel oder einem Halogenierungsmittel umgesetzt wird und die Verbindung der Formel (I) abgetrennt wird.

12. Herbicide Zusammensetzungen, welche als aktiven Bestandteil eine Verbindung nach einem der Ansprüche 1 bis 7 in Mischung mit einem Träger, bei dem es sich um ein festes oder flüssiges Verdünnungsmittel handelt, enthalten.

**Revendications**

1. Diphényléthers répondant à la formule (I):

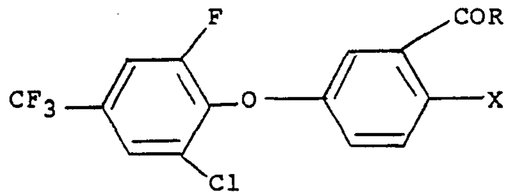

(I)

dans laquelle X est un groupe nitro ou un atome de chlore et R est:

(a) un groupe $ZR^3$ dans lequel Z est l'oxygène ou le soufre, et $R^3$ est un radical aliphatique ou alicyclique ayant jusqu'à 6 atomes de carbone portant un ou plusieurs des substituants suivants: halogéno; nitro; hydroxy; $CF_3$; alkoxy contenant 1 à 4 atomes de carbone; alkylthio, alkylsulfinyle ou alkylsulfonyle, ayant chacun 1 à 4 atomes de carbone; phényle; hétérocyclyle contenant 5 ou 6 atomes dans le noyau; un groupe $—NR^1R^2$ tel que défini ci-dessous; cyano; carboxy ou un de ses sels; alkoxycarbonyle ayant 2 à 5 atomes de carbone; un groupe $—CONR^1R^2$ dans lequel $—NR^1R^2$ est tel que défini ci-dessous; formyle; alcanoyle ayant, par exemple 2 à 5 atomes de carbone; ou bien un groupe 1-pyrazolyle, 1-imidazolyle, 1-(1,2,4-triazolyle) ou 2-(1,2,3-triazolyle) portant facultativement un ou plusieurs substituants halogéno, méthyle, méthoxy ou méthylthio.

(b) Un groupe $—NR^1R^2$ dans lequel $R^1$ est l'hydrogène, un radical aliphatique facultativement substitué ayant 1 à 6 atomes de carbone, ou un radical alicyclique facultativement substitué ayant 3 à 6 atomes de carbone, ou un radical phényle facultativement substitué, et $R^2$ a la même définition que $R^1$ mais peut en outre représenter un groupe hydroxy; alkoxy ayant 1 à 4 atomes de carbone; alkoxy ayant 1 à 4 atomes de carbone substitué avec un groupe phényle ou un groupe carboxy ou un de ses sels, esters ou amides; ou alkoxy ayant 1 à 4 atomes de carbone substitué avec un groupe amino, ou un groupe mono- ou dialkylamino dans lequel les groupes alkyle ont 1 à 4 atomes de carbone; ou dans lequel le groupe $—NR^1R^2$ est constitué par un noyau morpholine, pipéridine ou pyrrolidine portant facultativement un substituant méthyle, ou par un radical succinimido ou phtalimido, ou bien par un groupe de formule

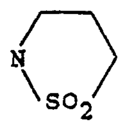

ou

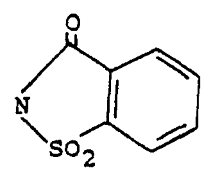

ou (c) un groupe $—NS^1S^2$ dans lequel $S^1$ est l'hydrogène, un groupe alkyle (par exemple alkyle en $C_1$ à $C_4$), un cation acceptable en agriculture, ou bien le chlore, le brome ou l'iode; et $S^2$ est un groupe

$$—\underset{\underset{O}{\|}}{C}OS^3$$

dans lequel $S^3$ est un radical aliphatique ou alicyclique facultativement substitué (ayant par exemple 1 à 6 atomes de carbone), ou un radical phényle facultativement substitué; ou $S^2$ est un groupe

$$—\overset{S^9\ \ S^4}{\overset{\diagdown\ \diagup}{(C)_n}}—S^5$$

dans lequel $S^4$ et $S^9$ représentent indépendamment l'hydrogène, un groupe alkyle en $C_1$ à $C_4$ ou un groupe phényle, et n a la valeur 1, 2 ou 3, et $S^5$ est un groupe CN, un groupe $—CONS^6S^7$ dans lequel chacun des substituants $S^6$ et $S^7$ est l'hydrogène, un radical aliphatique ou alicyclique (facultativement substitué (ayant par exemple 1 à 6 atomes de carbone) ou un radical phényle facultativement substitué; ou bien $S^5$ est un groupe

$$—\underset{\underset{O}{\|}}{C}OS^8$$

dans lequel $S^8$ est l'hydrogène, un cation acceptable en agriculture, ou un radical aliphatique ou alicyclique facultativement substitué (ayant par exemple 1 à 6 atomes de carbone), ou bien un radical phényle facultativement substitué.

ou

(d) un groupe 2-sulfonanoxy, phénylthio ou phénoxy facultativement substitué, ou un groupe —ON=$CR^4R^5$ dans lequel $R^4$ est l'hydrogène, un halogène, un groupe alkyle, aryle mononucléaire, alkoxy en $C_1$ à $C_6$, alkylthio en $C_1$ à $C_6$ ou dialkylamino; et $R^5$ est un groupe alkyle substitué ou non substitué, trifluorométhyle, cyano, carboxy, alkoxy substitué ou non substitué, alkoxycarbonyle, alkylcarbonyle, alkylthio, aminocarbonyle, arylcarbonyle mononucléaire, aryloxy mononucléaire ou carboxy, comprenant des amides, esters ou sels, acceptables en agriculture, dudit groupe carboxy; ou bien $R^1$ et $R^2$ peuvent être reliés entre eux pour former un noyau contenant 4 à 8 atomes de carbone.

2. Composé suivant la revendication 1, dans lequel, dans la formule (I), X est un groupe nitro ou un atome de chlore et R est (a) un groupe $ZR^3$, (b) un groupe $NR^1R^2$ ou (c) un groupe phénoxy, phénylthio, 2-nitrophénoxy ou 2-sulfolanoxy, ou bien un groupe —ON=$C(CH_3)_2$.

3. Composé suivant la revendication 1, dans lequel, dans la formule (I), X est un groupe nitro ou un atome de chlore et R est un groupe —$NS^1S^2$.

4. Composé suivant la revendication 1, dans lequel R est un groupe $ZR^3$ dans lequel Z est l'oxygène et $R^3$ est un radical alkyle ayant 1 à 6 atomes de carbone portant un ou plusieurs des substituants suivants: un atome d'halogène; un groupe nitro; un groupe carboxy ou un de ses sels ou esters alkyliques en $C_1$ à $C_6$; ou un groupe 1-pyrazolyle, 1-(1,2,4-triazolyle) ou 1-imidazolyle facultativement substitué avec le chore, un groupe méthyle ou méthoxy.

5. Composé suivant la revendication 1, dans lequel R est un groupe $ZR^3$ qui comprend un groupe phénoxy ou phénylthio, facultativement substitué avec un groupe nitro.

6. Composé suivant la revendication 1, dans lequel R est un groupe $NR^1R^2$ dans lequel $R^1$ est l'hydrogène ou un groupe alkyle ayant 1 à 6 atomes de carbone et $R^2$ est l'hydrogène, un groupe alkoxy ayant 1 à 6 atomes de carbone, ou un groupe alkoxy ayant 1 à 6 atomes de carbone substitué avec un groupe carboxy ou un de ses sels ou esters alkyliques en $C_1$ à $C_4$, ou bien dans lequel le groupe —$NR^1R^2$ est constitué d'un noyau morpholine, pipéridine ou pyrrolidine facultativement substitué avec un groupe méthyle, ou par un radical succinimido ou phtalimido, ou par un groupe de formule:

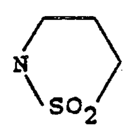

ou

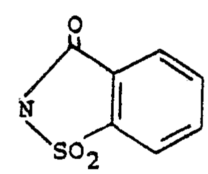

7. Composé suivant la revendication 3, dans lequel R est un groupe —$NS^1S^2$ dans lequel $S^1$ est l'hydrogène et $S^2$ est un groupe —$CHS^9S^5$ dans lequel $S^9$ est l'hydrogène ou un groupe alkyle en $C_1$ à $C_4$ et $S^5$ est un groupe —$CO.OS^8$ dans lequel $S^8$ est l'hydrogène, un cation ou un groupe alkyle en $C_1$ à $C_6$.

8. Composé suivant la revendication 1, dans lequel R est un groupe —ON=$CR^4R^5$ dans lequel $R^4$ est un groupe alkyle en $C_1$ à $C_4$ et $R^5$ est un groupe alkyle en $C_1$ à $C_4$ facultativement substitué avec un groupe (alkoxy en $C_1$ à $C_4$)-carbonyle.

9. Procédé pour inhiber la croissance de plantes indésirables, qui consiste à appliquer aux plantes, ou à leur milieu, une quantité phytotoxique d'un composé suivant l'une quelconque des revendications précédentes.

10. Procédé de préparation d'un composé de formule (I) suivant la revendication 1, qui consiste à faire réagir un composé de formule:

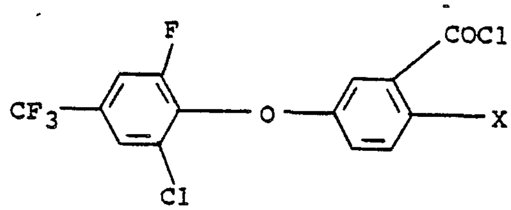

avec un composé de formule RH en présence d'un accepteur d'acide, et à recueillir le composé de formule (I).

11. Procédé de préparation d'un composé de formule (I) suivant la revendication 1, qui consiste à faire réagir un composé de formule:

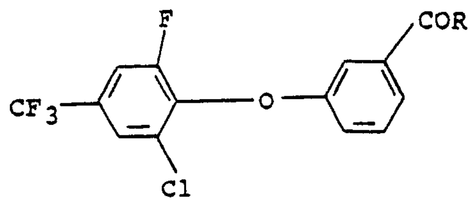

avec un agent de nitration ou d'halogénation, et à recueillir le composé de formule (I).

12. Compositions herbicides, comprenant comme ingrédient actif un composé suivant l'une quelconque des revendications 1 à 7, mélangé à un support comprenant un diluant solide ou liquide.